# EUROPEAN PATENT SPECIFICATION

(11) **EP 1 154 798 B1**
(45) Date of publication and mention of the grant of the patent: **10.05.2006**
(21) Application number: 00910711.1
(22) Date of filing: 24.02.2000
(51) Int. Cl.: A61K 47/48, A61K 51/10, A61P 35/00

(54) **MOLECULES FOR THE TREATMENT AND DIAGNOSIS OF TUMORS**
MOLEKÜLE FÜR DIE BEHANDLUNG UND DIAGNOSE VON TUMOREN
MOLECULES PERMETTANT DE TRAITER ET DE DIAGNOSTIQUER DES TUMEURS

(30) Priority: 24.02.1999 US 121340 P; 12.03.1999 EP 99200754
(43) Date of publication of application: 21.11.2001
(73) Proprietor: Universität Zürich, 8006 Zürich (CH)
(72) Inventor: ALBERTO, Roger, Ariel, CH-8400 Winterthur (CH); HÄFLIGER, Pascal, CH-1009 Pully (CH)
(74) Representative: Van Someren, Petronella F. H. M.
(86) International application number: PCT/EP2000/001553
(87) International publication number: WO 2000/050086

(56) References cited:
- EP-A- 0 879 606
- WO-A-93/21957
- US-A- 5 171 749
- US-A- 5 759 514
- BHALGAT M K ET AL: "Preparation and biodistribution of copper-67-labeled porphyrins and porphyrin -A6H immunoconjugates." NUCLEAR MEDICINE AND BIOLOGY, vol. 24, no. 2, February 1997 (1997-02), pages 179-185, XP000682959
- FAWWAZ, R. A. ET AL: "The use of a porphyrin bifunctional chelator for labeling of a monoclonal antibody with radioactive manganese" JOURNAL OF NUCLEAR MEDICINE: PROCEEDINGS OF THE 36TH ANNUAL MEETING, vol. 30, 1989, pages 935-936, XP000915982
- MERCER-SMITH J A ET AL: "The biodistribution of radiocopper-labeled compounds." ADVANCES IN EXPERIMENTAL MEDICINE AND BIOLOGY, (1989) 258 103-21. , vol. 258, 1989, pages 103-121, XP000916022
- LEWIS D ET AL: "The cytotoxicity of copper-67 porphyrin-antibody conjugates to a colon carcinoma cell line" ABSTRACTS OF PAPERS AMERICAN CHEMICAL SOCIETY, vol. 202, 25 - 30 August 1991, page Biot 168 XP000916021
- PIMM M V ET AL: "Biodistribution and tumour localisation of a daunomycin-monoclonal antibody conjugate in nude mice with human tumour xenografts." CANCER IMMUNOLOGY, IMMUNOTHERAPY , vol. 27, no. 3, 1988, pages 267-271, XP000915990
- TRAIL, P A: "Site directed delivery of anthracyclines for treatment of cancer" DRUG DEVELOPMENT RESEARCH, vol. 34, no. 2, February 1995 (1995-02), pages 196-209, XP000916019
- DATABASE WPI Week 199637, Derwent Publications Ltd., London, GB; Class B04, AN 1996-371135 'Detecting, localising and treating malignant tumours- using a pituitary adenylate cyclase-activating polypeptide (PACAP) cpd labelled with a radioisotope or paramagnetic metal' & WO 96 23527 A (MALLINCKRODT MEDICAL INC) 08 August 1996
- DATABASE WPI Week 199019, Derwent Publications Ltd., London, GB; Class A96, AN 1990-139986 'Metal radionuclide-labelled particulates compsn. used as dry aerosol for lung scintigraphy or radiotherapy of lung tumours' & WO 90 03803 A (MALLINCKRODT INC) 19 April 1990
- DATABASE WPI Week 198714, Derwent Publications Ltd., London, GB; Class B04, AN 1987-101135 'Radio-labelling antibodies with technetium and rhenium- in the presence of diethylene:tetra:amine penta:acetic acid' & US 4 652 440 A (PAIK C.H.) 24 March 1987

## Description

The present invention relates to new molecules for the treatment and diagnosis of tumors. The invention furthermore relates to therapeutical compositions comprising one or more of these molecules and to the use of both in treatment and diagnosis of cancer.

The diagnosis and therapy of cancer still requires a large input from the pharmaceutical and chemical industry. Although a substantial effort is made to develop new treatments, there are still many tumor types for which no treatment exists. An additional problem is the formation of micrometastases, which cannot be diagnosed or treated.

An important problem in treatment is the similarity between normal cells and cancer cells. Treatments interfering with the growth of tumor cells will also interfere in the growth of healthy cells. Radiotherapy as it is now known consists essentially of an arbitrary cross-fire from outside the cell or the cytoplasm. Because this is a rather rough treatment surrounding cells and tissues might also be damaged leading to more or less severe side effects.

The provision of an improved radiotherapy and diagnostic method for cancer which uses very low amounts of radionuclides and leads to a direct treatment in the malignant cell is therefore highly desirable.

It is known that the metabolism of cancer cells differs from that of normal cells. In addition, cancer cells appear to have an increased membrane permeability in comparison to normal cells due to an increased expression of membrane receptors. The result is that the cancer cells are more permeable for biological vectors, like proteins and peptides.

The enhanced uptake of such biological vectors can be used in the diagnosis of tumors by binding a radionuclide to a protein, for example by iodination of tyrosine functions in the protein or by covalent coupling of radioactive metal complexes. These molecules combine a tumor seeking function and a radioactive function. Although these types of molecules have been used for diagnosis, their use in therapy was not yet described.

It is the object of the present invention to further improve on the above described molecules to come to an even better tailored treatment of malignant cells.

This object is achieved by the invention by the provision of a molecule comprising an intercalating moiety which is coordinated to a radioactive [M(CO)₃]⁺-moiety, wherein M is a metal selected from ^{99m}Tc, ¹⁸⁶Re, ¹⁸⁸Re and Mn, and a tumor seeking molecule which is attached by direct linkage to a coordination site on the metal. The molecule can be targeted specifically to the tumor by the tumor seeking molecule and be internalized by the cell. The intercalating moiety will then insert into the DNA strand and induce breaks. In addition, the radioactive metal will also lead to strand breaking of the DNA. The advantage of the new molecules is that they are specifically directed to the malignant cell and are taken up by the cell.

The tumor seeking molecule is preferably a biomolecule, such as a peptide or protein that is actively targeted to the tumor cell. Examples of these biomolecules are somatostatin-, neurotensin-, bombesin-receptor binding molecules, monoclonal antibodies, penetratines^{™}, and glycoproteins, and molecules binding to the GPIIb/IIIa receptors. The invention is however not limited to these examples and is more generally applicable to other tumor seeking agents as well. This category encompasses in addition compounds which are known to be transported into the nucleus or the nucleus membrane. Examples of these are anti-sense oligonucleotides, proliferating agents, like deoxyuridine, and small molecules, like spermidine.

The intercalating moiety is preferably an aromatic molecule with an intercalative binding affinity for double-stranded DNA. Examples of such aromatic compounds are compounds containing i.e. acridine, porphyrin, ellipticine, phenantroline, carbazole, benzimidazole or compounds with known cytostatic activity (antibiotics) from the class of tetracyclines (anthracyclines), such as daunorubicine, epirubicine or mixoxantrone and are functionalized with ligands able to coordinate the [M(CO)₃]⁺ moiety. Examples of such ligands are those mentioned in EP-879 606 and additionally polyamino-polycarboxylates, phosphates and phosphonates, aliphatic or aromatic or mixed triamines and thiones.

The intercalating and tumor seeking functions are sometimes combined in existing molecules. Examples of intercalating agents combining an intercalating moiety and a peptide are actinomycin and triostin.

The radioactive molecule is selected from ^{99m}Tc, ¹⁸⁶Re, ¹⁸⁸Re and Mn. These pure γ-emitting nuclides are used since their accompanying low range conversion electrons will lead to cleavage of bonds, which are close to the decaying nucleus. The dose burden to the patient remains thus very low.

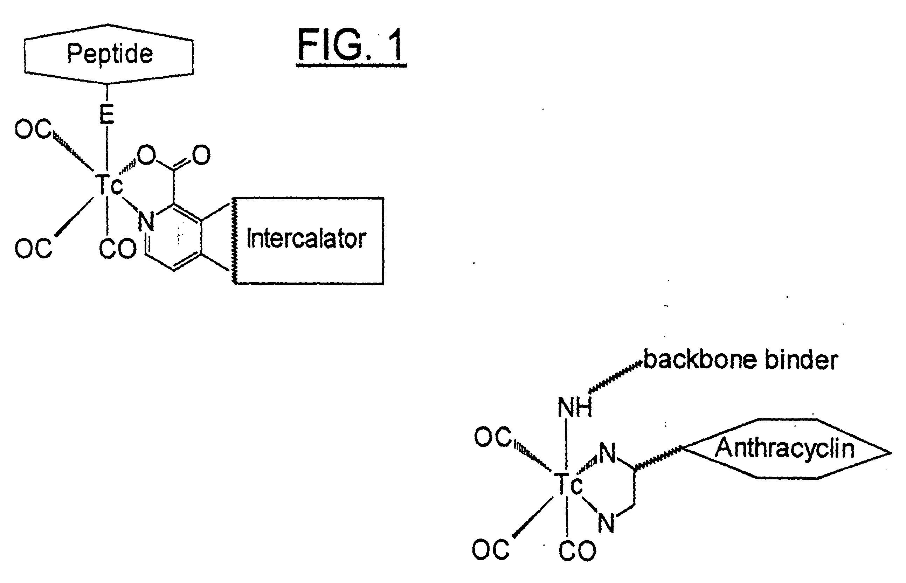

Particularly suitable combinations of the three functions are given in Fig. 1.

The invention further relates to the use of the molecules in therapy and diagnosis and to therapeutical and diagnostic compositions comprising one or more of these molecules.

Therapeutical compositions comprise at least a suitable amount of the molecule in a diluent or excipient. Such compositions can take the form of solutions and are administered intravenously, intraperitoneally or intrathecally. Suitable amounts to be administered depend on the way of administration, the radionuclide used and the indication to be treated or diagnosed. Suitable amounts vary between 10⁻⁹ and 10⁻¹ g per kg body weight.

Excipients and diluents for this type of medication are well known to the skilled person. However, the present molecules require certain conditions for stability. Preferably, the excipient or diluent should be of a hydrophilic and preferably organic nature.

For diagnostic purposes the composition consists of at least a suitable amount of the molecule in a diluent or excipient. Diagnostic methods to be used with the composition of the invention are scintigraphy or Magnetic Resonance Imaging (MRI).

It was now found that the method for the synthesis of Tc and Re carbonyls from water described in EP-879 606 is suitable for preparation of the molecules of the invention. It is in particular possible with this method to introduce intercalating ligands, which form very stable complexes (in vitro and in vivo) with the above mentioned carbonyls.

The ligands claimed in EP-879 606 and acridine, porphyrin, ellipticine, phenantroline, carbazole, benzimidazole do stabilize the fac-[Tc(CO)₃]⁺ moiety in serum and form complexes at very low concentrations. These ligands can be site specifically attached to the biomolecules and subsequently be labeled with i.e. Tc-99m. Since the radionuclide is very close to the intercalating ligand, its low energy electron will penetrate the DNA-strands very well and induce strandbreaking. When intercalating in one of the grooves, the probability to hit is very high since the nucleus is practically surrounded by DNA.

The biomolecules derivatized according to the invention exhibit high selectivity and are internalized. As known from pure organic intercalators, the complex is going to intercalate in DNA in particular when the cell is dividing. In contrast with other therapeutics, a high selectivity can be achieved with this combination.

If Re-188 is applied as the radionuclide, the damages will be much more severe than in the case of Tc-99m, but, consequently, the applied amount of radioactivity will be much lower than in case of "normal" radiotherapy. Thus, severe side effects such as bone marrow toxicity could be avoided.

The present invention will be further illustrated in the examples that follow and which are solely intended to clarify the invention, but are in no way intended to be limiting to the scope thereof.

In the Examples reference is made to the following figures:
**Figure 1:** schematic representation of molecules of the invention.
**Figure 2**: example of a Tc(I) complex with this intercalating ligand and a potential biomolecule attached by direct linkage to another coordination site.
**Figure 3**: schematic representation of the method for preparing molecules of the invention.
**Figure 4:** schematic representation of the three types of plasmid structures.
**Figure 5:** ethidium bromide stained agarose gel of the three types I, II and III of DNA (left lane) and a molecular weight marker (right lane).
**Figure 6**: ethidium bromide stained agarose gel of a plasmid preparation treated with the compound [^{99m}Tc(P₁)(teta)(CO)₃]; The application site of the sample is on the bottom of the gel. Lane 1 is the molecular weight marker; lane 2 is a reference solution containing supercoiled, relaxed (single strand break) and linearized (double strand break) plasmid, lane 3 is the experimental solution containing both plasmid and the intercalator of the invention, and lane 4 is the negative reference containing only plasmid.
**Figure 7**: reaction scheme for the preparation of model bifunctional intercalators.
**Figure 8**: reaction scheme for the preparation of model trifunctional intercalators.

### EXAMPLES

### EXAMPLE 1

### Synthesis of the molecules of the invention

### 1. Introduction

To provide a strong intercalation, the intercalator should be preferably planar and aromatic heterocyclic. Furthermore, pendant groups in the intercalator must stably be coordinated to the radionuclide (i.e. ^{99m}Tc). In this example, it is not coercive that the coordinating unit must be a multidentate ligand with high thermodynamic stability, since most complexes with Tc(I) show an extremely high kinetic stability. For these reasons and due to the already known principles of complexation of several mono- and bidentate ligands (especially picolinic acid) 5,6-benzochinolin-3-carboxylic acid was selected as intercalator.

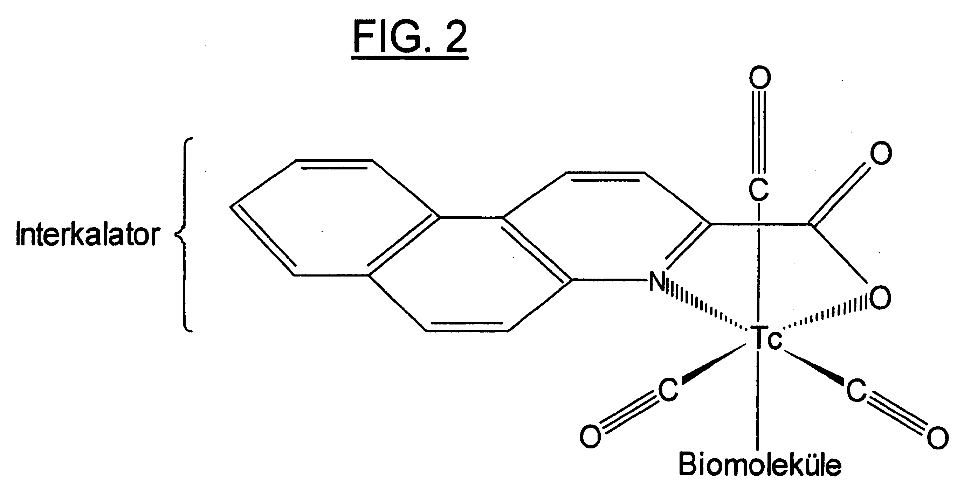

Figure 2 depicts an example of a Tc(I) complex with this intercalating ligand and a potential biomolecule attached by direct linkage to another coordination site.

### 2. Synthesis of the example intercalator

### 2.1. 3-cyano-4-benzoyl-3,4-dihydrobenzo(f)chinoline 2

648 µl (5.58 mmol) benzoyl chloride was added to a two phase system of water/methylene chloride over a period of two hours. These two layers contain 500 mg (2.79 mmol) of benzo(f)chinolin in the methylene chloride layer and 545 mg (8.37 mmol) KCN in water. Stirring was continued for 6 hours. The organic phase was separated and washed with water, 5% hydrochloric acid, water, 5% NaOH solution, and again with water. After drying over magnesium sulfate, the solution was evaporated to dryness.

The bromide salt of this so-called Reissert-compound was recrystallized from 95% ethanol to yield the analytically pure substance. Yield: 612 mg (71%).

### 2.2 5,6-benzochinolin-3-carbon acid (P1)

2 ml 48% hydrobromide acid were added to 287 mg (0.93 mmol) of the Reissert-compound dissolved in 2 ml acetic acid. The solution was refluxed during 24 hours, cooled and filtered. The filtered product was washed with diethyl ether, dried, and recrystallized from methanol to yield 169 mg (0.76 mmol) (82%) of the hydrobromide of the intercalator as a yellow solid.

### 2.3 Macroscopic synthesis of Technetium and Rhenium complexes with P1 (5,6-benzochinolin-3-carbon acid)

### 2.3.1 [NEt₄][ReBr(P1)(CO)₃]

A suspension of 102 mg (133 µmol) [NEt₄][ReBr₃(CO)₃], 29.7 mg (133 µmol) P1 and 116 µl (226 mmol) of trioctylamine were refluxed in dichloromethane until a clear solution was achieved. After evaporation of the solution, the complex 5 was extracted into THF. After evaporization of THF the residue was washed with diethyl ether to remove trioctyl ammonium bromide. Yield: 63 mg (67%) of the yellow complex.

### 2.3.2 [Re(P₁)(H₂O)(CO)₃]

200.0 mg (0.26 µmol) of [NEt₄]₂[ReBr₃(CO)₃] were refluxed in the presence of 29.1 mg of the intercalator P1 during 4 hours in 1M MES-buffer solution. Then the yellow precipitation was filtered. Yield: 114.2 mg (86%).

### 2.4 Microscopic synthesis of [^{99m}Tc(H₂O)(P₁)(CO)₃]

The ^{99m}Tc complexes were synthesized in a two-step procedure with a normal generator eluate. In a first step the complex was synthesized in >97% yield according to the literature (R. Alberto et al., J. Am. Chem. Soc. 120, 7987 (1998)). The solution was then neutralized with phosphate buffer in the reaction vial and a solution of the corresponding ligand was added. The end concentration was between 10⁻⁴ and 10⁻⁵. It was left standing for 30 minutes at 75°C. The radio-chemical purification and the yield were defined through HPLC-chromatography and it was discovered that [^{99m}Tc(HPO₄)(P₁)(CO)₃]²⁻ (compound **10**) with a yield of 80-95% (dependent on the ligand concentration and the reaction time) was formed.

### 2.5 Synthesis of model trifunctional molecules of the invention

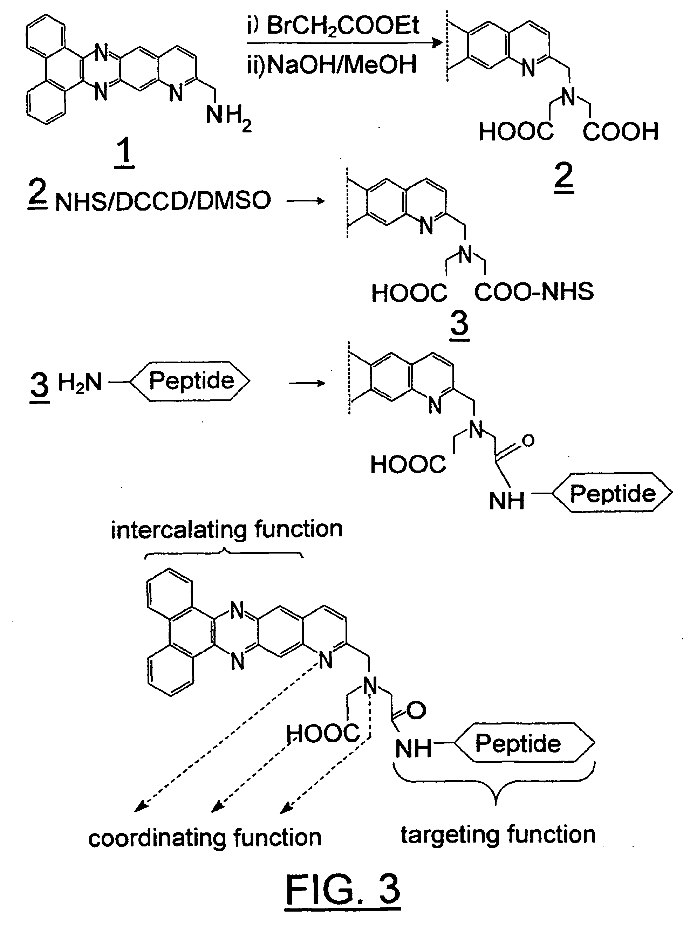

This is an example how a trifunctional molecule can be built. The procedure is based on known synthetic approaches for the corresponding coupling methods. The schematic procedure is given in Figure 3.

### 1. Syntheses of the bifunctional ligands, bearing an intercalator and a coordinating functionality

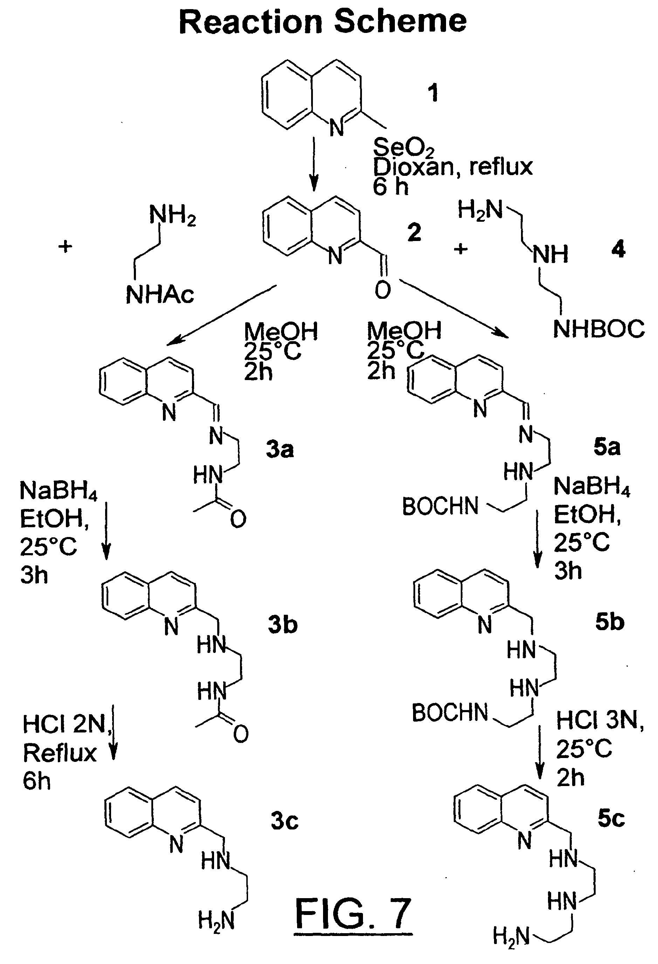

A bifunctional ligand was prepared according to the strategy described in Fig. 3. Fig. 7 gives the specific reaction scheme of the reaction that is described hereinbelow.

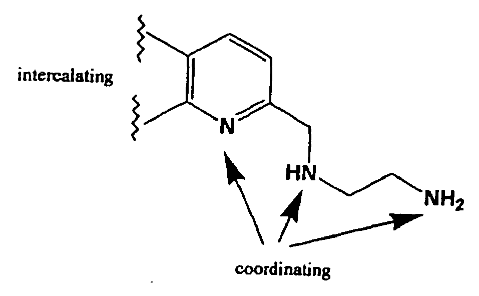

### 2-Methylquinoline (1)

2-Methylquinoline **(1)** was bought from Fluka and used without further purification.

### Quinoline-2-carbaldehyde (2)

A mixture of 5.5 g of selenium dioxyde (49.5 mmol) in 50 ml dioxane and 2 ml water was added in small portions over 10 minutes to a boiling solution of 4.4 g (30.7 mmol) of 2-methylquinoline (**1**) in 20 ml dioxane. After 6 hours of boiling, the warm reaction mixture was filtered. The filtrate was evaporated, dissolved in dichloromethane and filtered through Alox. The yellow-brown solid product obtained after evaporation of the solvent was recrystallized from dichlormethane. Yield: 3.76 g (78%)
¹H-NMR (DMSO):δ, 10.12s, 8.61d, 8.22d, 8.12d, 7.99d, 7.91t, 7.79t

### Compound 3a

A mixture of 500 mg of quinoline-2-carbaldehyde (2) (3.2 mmol) and 330mg of N-(2-aminoethyl)-acetamid (3.23 mmol) in 15 ml of methanol was stirred for 2 hours at room temperature. The light brown solid product obtained was directly used for the next reaction. Yield: ~770 mg (-100%)
¹H-NMR (CDCl₃):δ, 8.57s, 8.21d, 8.13d, 8.10d, 7.85d, 7,75t, 7.59t

### Compound 3b

A solution of 175 mg (4.62 mmol) of NaBH₄ in 10 ml of ethanol was slowly added over 2 hours to a stirred solution of 500 mg (2.07mmol) of **3a** in 30 ml ethanol at 0°C. This mixture was then stirred overnight at room temperature. The solid substance obtained after evaporation of the solvent was triturated with a 3M Na₂CO₃ solution. The desired light brown product (**3b**) was then extracted with dichloromethane. Yield: 382 mg (76%)
¹H-NMR (CDCl₃):δ, 8.15d, 8.05d, 7.81d, 7.71t, 7.54t, 7.35d, 6.84br, 4.21s, 3.50q, 3.02t, 2.02s

### Compound 3c

A solution of 200 mg of **3b** (0.82 mmol) in 20 ml of 2N HCl was refluxed for 6 hours. The oil obtained after evaporation of the solvent was washed with ethanol to give the desired light brown solid hydrochloride salt **3c**. Yield: 203 mg (90%)
¹H-NMR (D₂O):δ, 8.40d, 7.95t, 7.76t, 7.59t, 7.49d, 4.57s, 3.46t, 3.34t

### N-BOC-diethylentriamine (4)

A solution of 500mg (2.29 mmol) of di-tert-butyl dicarbonate ((BOC)₂O) in 30 ml dioxan was slowly added to a solution of 1.49 ml (1.42 g) (13.74 mmol) of diethylentriamine in 80 ml of dioxan at 10°C. The mixture was then stirred for 15 hours at room temperature. The desired product precipitated as an oil, which was then separated from the rest of the solution, dissolved in water, filtered, and extracted with dichloromethane to finally give the desired product as a light yellow oil. Yield: 260 mg (56%)
¹H-NMR (CDCl₃): δ, 5.15br, 3.25br, 3.18t, 2.77t, 2.69t, 2.63t, 1.76br, 1.41s, 1.19t

### Compound 5a

A mixture of 140 mg of quinoline-2-carbaldehyde (2) (0.89 mmol) and 200mg of N-BOC-diethylentriamine (0.99 mmol) in 30 ml of methanol was stirred for 3 hours at room temperature. The solid obtained after evaporation of the solvent was then washed with water to obtain the desired light brown product. Yield: 304 mg (94%)
¹H-NMR (DMSO):δ, 8.32d, 7.97t, 7.73t, 7.71d, 7.57t, 6.65t, 4.33s, 3.08t, 2.97t, 2.85t, 1.28s, 1.09t

### Compound 5b

A solution of 41 mg (1.08 mmol) of NaBH₄ in 10 ml of ethanol was slowly added over 2 hours to a stirred solution of 148 mg (0.43mmol) **5a** in 30 ml of ethanol at 0°C. This mixture was then stirred overnight at room temperature. The solid brown oil obtained after evaporation of the solvent was triturated with a 3M Na₂CO₃ solution. The desired light brown product (**3b**) was then extracted with dichloromethane. Yield: 136 mg (92%)
¹H-NMR (DMSO):δ, 8.29d, 7.94d, 7.92d, 7.71t, 7.61d, 7.54t, 6.71t, 3.95s, 2.96q, 2.59s, 1.33s, 1.22t

### Compound 5c

A solution of 100 mg of **5b** (0.29 mmol) in 3N HC1 was refluxed for 2 hours. The oil obtained after evaporation of the solvent was washed with diethylether to give the desired light brown solid hydrochloride salt **5c.** Yield: 102 mg (94%)
¹H-NMR (D₂O):δ, 8.44d, 7.95t, 7.77t, 7.6t, 7.51d, 4.51s, 3.44s, 3.34t, 3.27t

### 2. Synthesis of trifunctional model intercalators

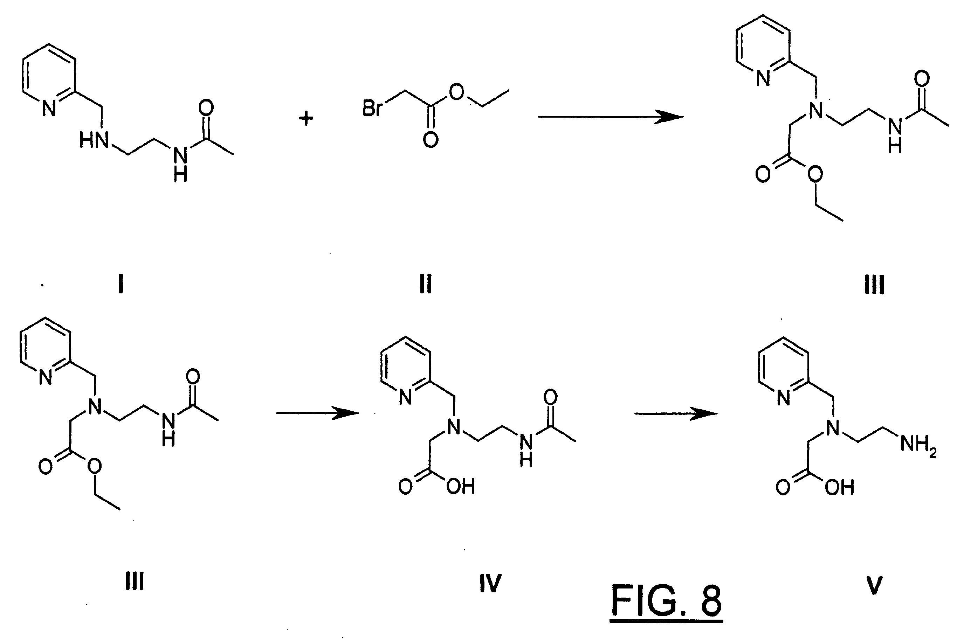

Trifunctional intercalators were prepared starting from **5a** or **3b** of part 1 above. Fig. 8 gives the specific reaction scheme.

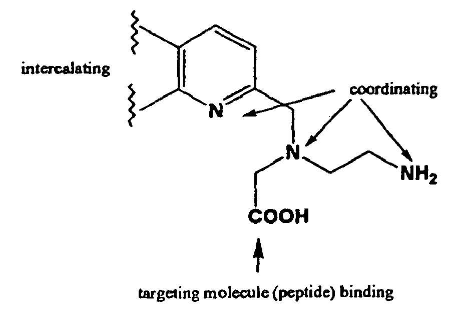

### 1. alkylation of an amine with bromo-aceticacidethylester

Amine **I** (Fig. 7; 547 mg, 2.83 mmol) and triethylene amine (0.510 ml, 3.08 mmol) were stirred in methanol (10 ml). The solution was cooled to 0°C, and ethyl bromoacetate **II** (0.313 ml, 2.83 mmol) was added dropwise within 5 minutes. After stirring the solution at room temperature for 18 hours, the solvent was removed in vacuo. The residue was dissolved in dichloromethane (50 ml) and washed three times with water (20 ml). The water phases were washed twice with dichloromethane (50 ml). The organic phases were dried over MgSO₄, filtered, and the solvent was removed in vacuo to give III as a yellow oil. Yield: 590 mg (2.11 mmol, 74.6%).
TLC (silica, ethanol) R_{F} 0.4
¹H NMR (200 MHz, d₆-acetone) δ = 8.44 (m, 1H, picolin), 7.65 (m, 1H, picolin), 7.45 (m, 1H, picolin), 7.21 (m, 1H, picolin), 4.12 (q, 2 H, J = 7.2 Hz, CH₂ ester), 3.94 (s, 2H, CH₂), 3.64 (s, 2H, CH₂), 3.32 (m, 2H, N-CH₂-CH₂-N), 2.82 (m, 2H, N-CH₂-CH₂-N), 1.84 (s, 3H, CH₃-CO), 1.21 (t, 3H, J = 7.2 Hz, CH₃ ester).

### 2. deprotection

Amine III (576 mg, 1.94 mmol) was dissolved ethanol (4 ml) and water (8 ml). NaOH 2M (2 ml) was added, and the solution was stirred at room temperature for 1.5 hours. Analytical HPLC exhibited a single peak, indicating that the ester group was cleaved quantitatively.

The solvent was removed in vacuo, the residue was dissolved in water (8 ml), and HC1 2N (1 ml) was added to neutralize the solution. HC1 33% (1.0 ml) was added, and the reaction mixture was stirred at 90°C for 48 hours. NaHCO₃ was added to neutralize the reaction mixture, the solvent was removed in vacuo and the residue was washed with ethanol. Removing of the solvent gave the deprotected product V as a yellow oil. Yield: 352 mmol (1.68 mmol, 68.6%).
¹H NMR (300 MHz, D₂O) δ = 8.44 (m, 1H, picolin), 7.85 (m, 1H, picolin), 7.45 (m, 1H, picolin), 7.39 (m, 1H, picolin), 3.78 (s, 2H, CH₂), 3.35 (m, 2H, N-CH₂-CH₂-N),3.22 (s, 2H, CH₂), 3.32), 2.79 (m, 2H, N-CH₂-CH₂-N).

### EXAMPLE 2

### Strand breaking with the molecules of the invention in a model system

### 1. Introduction

### 1.1 The use of plasmids

To investigate the ability of the intercalating complexes with ^{99m}Tc to induce DNA-strandbreaks, plasmids were used as a model system. Plasmids are very suitable because electrophoretic analyses allow to differentiate between double and single strand breaks. Additionally, large quantities of plasmids can be produced very simply by using cell biological methods.

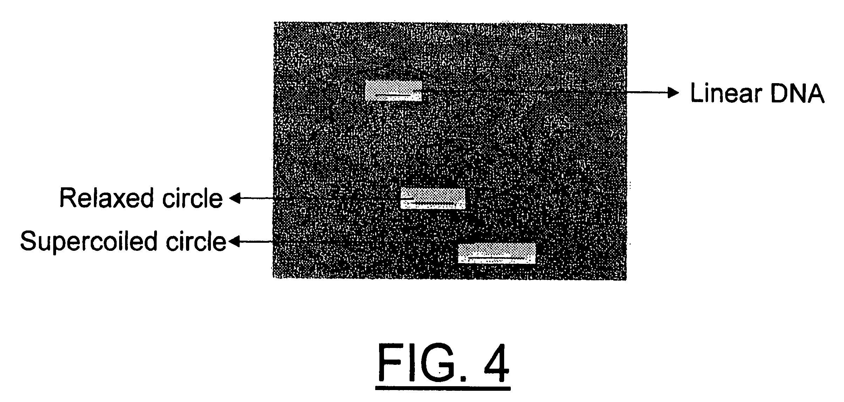

A plasmid is a circular double-stranded DNA molecule, which double helical axis can be drilled into a superhelix. This form of the superhelix is described as type I. This type may loose its superhelix-structure by a single strandbreak and is then present as a relaxed circular DNA (type II). Through a double strandbreak of both types a linear from (type III) of the plasmid will be created. Figure 4 shows an example of the structure of these 3 DNA types.

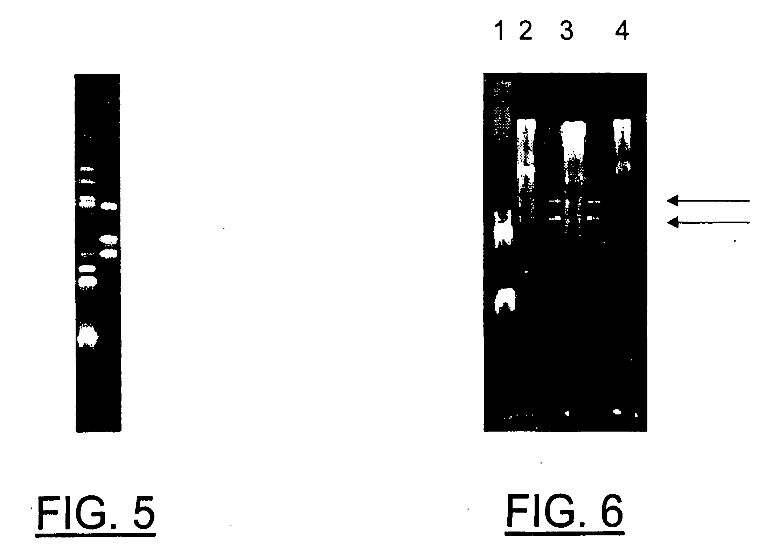

Because these three DNA types have different structures, they may well be separated due to their size and especially their form by electrophoresis on agarose gel. The mixture (type I-III after the experiment) to be investigated is loaded on an agarose gel. A constant voltage will then be applied and the negatively charged DNA-fragments will migrate toward the cathode. The larger the form of the fragment, the slower the migration along the gel. DNA of type I (most compact) moves fastest, type II slowest. The gel will then be put in solution which contains very little ethidium bromide. The DNA fragments are made visible by intercalation and irradiation with UV-light of 300 nm depicting red-orange colored fluorescence (590 nm). This method is so sensitive that less than 5 ng DNA per band are detected. In the photographic record of the gels in Figure 5, the migration direction is from the top to the bottom.

### 1.2 Production of the plasmids

The plasmid Bluescript KS^{™} with a size of 2958 base pairs has been produced following the standard protocol of the company QIAGEN. Usually, this plasmid exists in the superhelix form (type I). With the restriction enzyme KpnI the linearized form of the plasmid DNA (type III) can be produced. A single strandbreak resulting in the relaxed circular form of plasmid (type II), can be induced by the enzyme DNAase I. Figure 5, right lane, shows the electrophoresis on agarose-gel of a mixture of these three types of DNA. For the electrophoresis a marker with several sizes of DNA-pieces has been used as reference (left lane).

As demonstrated by the three bands of the right lane, the three types of DNA were clearly separated and can be distinguished after visualization. If single or double strand breaks result from conversion electrons, it should be easy detectable by this method.

### 2. Investigation of the ability of [^{99m}Tc(P₁) (teta) (CO)₃] to induce strandbreaks

5 µl of a solution containing approx. 0.3 mCi/ ml of [^{99m}Tc(P₁) (teta) (CO)₃] and 100 ng of type I plasmid (~=3*10⁻⁵ M in base pairs) were left standing over a period of 18 hours. Then a electrophoresis of this mixture and the three references was made (Figure 6).

It is clearly visible that the plasmids in the measurement solution (lane 3) migrate slower than in the reference solution. The reason for this observation is that a small change in the structure of the plasmids is probably induced by intercalation of the complex into the double strand. This change in the tertiary structure of the plasmid did then allow a better intercalation of the ethidium bromide, thus explaining the stronger intensity of the band of sample solution.

Furthermore, in the comparison with the negative reference solution (lane 4), it is obvious that one or possibly two new bands appeared (arrows) in the lane of the solution treated with the ^{99m}Tc complex. The stronger of these two bands corresponds approximately to the position of type II on the band of reference solution in lane 2, containing the three types. This means that the complex has induced a single strand break in the plasmid.

## Claims

1. Molecules for treatment or diagnosis of tumors and malignancies, comprising an intercalating moiety which is coordinated to a radioactive [M(CO)₃]⁺-moiety, wherein M is a metal selected from ^{99m}Tc, ¹⁸⁶Re, ¹⁸⁸Re and Mn, and a tumor seeking biomolecule which is attached by direct linkage to a coordination site on the metal.

2. Molecules as claimed in claim 1 wherein the biomolecule is selected from the group consisting of somatostatin-, neurotensin-, bombesin-receptor binding 10 molecules, antibodies, penetratines, and molecules binding to the GPIIb/IIIa receptors.

3. Molecules as claimed in claims 1 and 2 wherein the intercalating agent is an aromatic molecule with an intercalative binding affinity for doublestranded DNA. 15

4. Molecules as claimed in claim 3, wherein the intercalating agent is selected from the group consisting of acridine, porphyrin, ellipticine, phenantroline, carbazole, benzimidazole or compounds with known cytostatic activity (antibiotics) from the class of tetracyclines (anthracyclines), such as daunorubicine, epirubicine or mixoxantrone.

5. Molecules as claimed in claims 1-4 having the general structural formula as given in Fig. 2.

6. Molecule as claimed in claims 1-5, having any one 25 of the structures as shown in Fig. 1.

7. Molecules as claimed in claims 1-6 for use as or in a therapeutic or diagnostic agent for treating or diagnosing tumors or malignancies.

8. Use of molecules as claimed in claims 1-6 for the 30 preparation of a therapeutic or diagnostic agent for treating or diagnosing cancer tumors or malignancies.

9. Therapeutical composition, comprising one or more molecules as claimed in claims 1-6 and one or more suitable excipients. 35

## Patentansprüche

1. Moleküle zur Behandlung oder Diagnose von Tumoren and bösartigen Tumoren, die eine interkalierende Gruppe, die an eine radioaktive [M(CO)₃]⁺-Gruppe koordiniert ist, wobei M ein Metall ausgewählt aus ^{99m}Tc, ¹⁸⁶Re, ¹⁸⁸Re und Mn ist, und ein Tumor suchendes Biomolekül umfassen, das durch eine direkte Bindung mit einer Koordinationsstellen an dem Metal verbunden ist.

2. Moleküle gemäß Anspruch 1, wobei das Biomolekül ausgewählt ist, aus der Gruppe bestehend aus Somatostatin-, Neurotensin-, Bombesin-rezeptorbindenden Molekülen, Antikörpern, Penetratinen und Molekülen, die an GPIIb/IIIa-Rezeptoren binden.

3. Moleküle gemäß Ansprüchen 1 und 2, wobei das interkalierende Mittel ein aromatisches Molekül mit einer interkalativen Bindungsaffinität für doppelsträngige DNA ist.

4. Moleküle gemäß Anspruch 3, wobei das interkalierende Mittel ausgewählt ist aus der Gruppe bestehend aus Acridin, Porphyrin, Ellipticin, Phenantrolin, Carbazol, Benzimidazol oder Verbindungen mit bekannter Zytostatischer Aktivität (Antibiotika) aus der Klasse der Tetracycline (Anthracycline), wie etwa Daunorubicin, Epirubicin oder Mixoxantron.

5. Moleküle gemäß Ansprüchen 1 bis 4, welche die in Fig. 2 gezeigte allgemeine Strukturformel aufweisen.

6. Moleküle gemäß Ansprüchen 1 bis 5, die eine der in Fig. 1 gezeigten Strukturen aufweisen.

7. Moleküle gemäß Ansprüchen 1 bis 6 für die Verwendung als oder in einem therapeutischen oder diagnostischen Mittel zur Behandlung oder Diagnose von Tumoren oder bösartigen Tumoren.

8. Verwendung von Molekülen gemäß Ansprüchen 1 bis 6 für die Herstellung eines therapeutischen oder diagnostischen Mittels zur Behandlung oder Diagnose von Krebstumoren oder bösartigen Tumoren.

9. Therapeutische Zusammensetzung, die eines oder mehrere der Moleküle gemäß Ansprüchen 1 bis 6 und einen oder mehrere geeignete Hilfsstoffe umfasst.

## Revendications

1. Molécules pour le traitement ou le diagnostic des tumeurs et des affections malignes, comprenant un résidu intercalant qui est lié par coordination à un résidu [M(CO)₃]⁺ radioactif, dans lesquelles M est un métal choisi parmi ⁹⁹mT_{c}, ¹⁸⁶Re, ¹⁸⁸Re et Mn et une biomolécule chercheuse de tumeurs qui est liée par liaison directe à un site de coordination sur le métal.

2. Molécules selon la revendication 1, dans lesquelles la biomolécule est choisie dans le groupe constitué par les molécules de liaison au récepteur de la somatostatine, de la neurotensine, de la bombésine, les anticorps, les pénétratines, et les molécules de liaison aux récepteurs GPIIb/IIIa.

3. Molécules selon les revendications 1 et 2, dans lesquelles l'agent intercalant est une molécule aromatique ayant une affinité de liaison à caractère intercalant pour l'ADN double brin.

4. Molécules selon la revendication 3, dans lesquelles l'agent intercalant est choisi dans le groupe constitué par l'acridine, la porphyrine, l'ellipticine, la phénantroline, le carbazole, le benzimidazole ou les composés ayant une activité cytostatique connue (antibiotiques) issus de la classe des tétracyclines (anthracyclines), tels que la daunorubicine, l'épirubicine ou la mixoxantrone.

5. Molécules selon les revendications 1 à 4 ayant la formule développée générale indiquée à la Figure 2.

6. Molécules selon les revendications 1 à 5, ayant l'une des formules développées indiquées à la Figure 1.

7. Molécules selon les revendications 1 à 6 pour utilisation comme telle ou dans un agent thérapeutique ou diagnostique pour le traitement ou le diagnostic des tumeurs ou des affections malignes.

8. Utilisation de molécules selon les revendications 1 à 6 pour la préparation d'un agent thérapeutique ou diagnostique pour le traitement ou le diagnostic des tumeurs cancéreuses ou des affections malignes.

9. Composition thérapeutique, comprenant une ou plusieurs molécules selon les revendications 1 à 6, et un ou plusieurs excipients appropriés.
